# EUROPEAN PATENT SPECIFICATION

(11) **EP 4 125 905 B1**
(45) Date of publication and mention of the grant of the patent: **25.12.2024**
(21) Application number: 20730707.5
(22) Date of filing: 02.04.2020
(51) Int. Cl.: A61K 31/4985, A61K 45/06, A61P 15/00, A61P 31/04

(54) **REGIMEN FOR TREATING A NEISSERIA GONORRHOEAE INFECTION WITH GEPOTIDACIN**
SCHEMA ZUR BEHANDLUNG EINER NEISSERIA GONORRHOEAE-INFEKTION MIT GEPOTIDACIN
POSOLOGIE POUR TRAITER UNE INFECTION À NEISSERIA GONORRHOEAE AVEC DE LA GÉPOTIDACINE

(43) Date of publication of application: 08.02.2023
(73) Proprietor: GlaxoSmithKline Intellectual Property Development Limited, Stevenage SG1 2NY (GB)
(72) Inventor: SCANGARELLA-OMAN, Nicole, Collegeville, PA 19426 (US); DUMONT, Etienne, Collegeville, PA 19426 (US); BERGESCH BARTH, Aline, Collegeville, PA 19426 (US)
(74) Representative: Fletcher, Rodney Justin
(86) International application number: PCT/IB2020/000253
(87) International publication number: WO 2021/198715

(56) References cited:
- STEPHANIE N TAYLOR ET AL: "Gepotidacin for the Treatment of Uncomplicated Urogenital Gonorrhea: A Phase 2, Randomized, Dose-Ranging, Single-Oral Dose Evaluation", CLINICAL INFECTIOUS DISEASES, vol. 67, no. 4, 2 April 2018 (2018-04-02), US, pages 504 - 512, XP055715877, ISSN: 1058-4838, DOI: 10.1093/cid/ciy145
- ANONYMOUS: "History of Changes for Study: NCT04010539 A Study Evaluating Efficacy and Safety of Gepotidacin Compared With Ceftriaxone Plus Azithromycin in the Treatment Urogenital Gonorrhea", CLINICALTRIALS.GOV ARCHIVE, 27 March 2020 (2020-03-27), XP055752422, Retrieved from the Internet <URL:https://www.clinicaltrials.gov/ct2/history/NCT04010539?V_7=View#StudyPageTop> [retrieved on 20201119]
- ANONYMOUS: "History of Changes for Study: NCT04079790 Pharmacokinetics of Gepotidacin Tablets in Adults and Adolescents Subjects", CLINICALTRIALS.GOV ARCHIVE, 12 February 2020 (2020-02-12), pages 1 - 10, XP055752498, Retrieved from the Internet <URL:https://clinicaltrials.gov/ct2/history/NCT04079790?V_2=View#StudyPageTop> [retrieved on 20201119]
- ANONYMOUS: "GSK starts a phase III clinical programme for a potential first-in-class antibiotic, gepotidacin", 28 October 2019 (2019-10-28), pages 1 - 9, XP055752503, Retrieved from the Internet <URL:https://www.gsk.com/en-gb/media/press-releases/gsk-starts-a-phase-iii-clinical-programme-for-a-potential-first-in-class-antibiotic-gepotidacin/> [retrieved on 20201119]
- BRIAN D. VANSCOY ET AL: "Relationship between Gepotidacin Exposure and Prevention of On-Therapy Resistance Amplification in a Neisseria gonorrhoeae Hollow-Fiber In Vitro Infection Model", ANTIMICROBIAL AGENTS AND CHEMOTHERAPY, vol. 64, no. 10, 13 July 2020 (2020-07-13), US, pages 1 - 9, XP055752531, ISSN: 0066-4804, DOI: 10.1128/AAC.00521-20

## Description

This invention was made with government support under the United States of America Department of Health and Human Services Assistant Secretary for Preparedness and Response, Biomedical Advanced Research and Development Authority (BARDA), within the Office of the Assistant Secretary for Preparedness and Response in the U.S. Department of Health and Human Services agreement no.: HHSO100201300011C. The government has certain rights in this invention.

### FIELD OF THE INVENTION

The present invention relates to a treatment method for the treatment of an infection caused by *Neisseria gonorrhoeae,* comprising administering gepotidacin or a pharmaceutically acceptable salt thereof to a human in need thereof, in accordance with a treatment regimen as defined in the claims.

### BACKGROUND TO THE INVENTION

*Neisseria gonorrhoeae* (NG) is gram negative bacteria that can infect male and female humans. Infections are most common in the urogenital tract (including the cervix, uterus, and fallopian tubes in women, and the urethra in women and men) - generally called "gonorrhoea" - but can also be anorectal, conjunctival, or pharyngeal. If left untreated, infections caused by NG can disseminate to other areas of the body, which commonly causes synovium and skin infections. Consequences of untreated gonococcal infections include pelvic inflammatory disease, infertility in women and men, ectopic pregnancy, tubo-ovarian abscess, neonatal conjunctivitis, and disseminated gonorrhoea.

Over the past few decades, NG has demonstrated the ability to develop resistance to most antibiotics recommended or used for treatment, suggesting the possibility of untreatable gonorrhea in the future. The Centers for Disease Control and Prevention and the World Health Organization have labeled drug-resistant NG with threat levels of urgent and high, respectively, and identified a critical need for new antibiotic treatments.

Treatment failures with cefixime or other oral cephalosporins have been reported in Asia, Europe, South Africa, and Canada. Ceftriaxone, in combination with azithromycin, is the last line of treatment recommended by CDC, EU and AU guidelines and treatment failures for pharyngeal infections have been reported in Australia, Japan, UK and Europe. Most gonococcal infections of the pharynx (site of resistance and decreased efficacy that also serves as a reservoir for contamination of other anatomical sites - see for example Pharyngeal Clinical Infectious Diseases, Volume 49, Issue 12, 15 December 2009, Pages 1798-1800, https://doi.org/10.1086/648428) are asymptomatic. Gonococcal infections of the pharynx are more difficult to eradicate than are infections at urogenital and anorectal sites. Few antimicrobial regimens, including those involving oral cephalosporins, can

reliably cure >90% of gonococcal pharyngeal infections. New oral antibiotics with favourable penetration properties at the pharyngeal site are urgently needed to address this major public health threat.

Gepotidacin is a novel triazaacenaphthylene bacterial type II topoisomerase inhibitor that is currently in development. WO2016/027249 discloses generally the use of certain triazaacenaphthylene bacterial type II topoisomerase inhibitors, including gepotidacin, for the use in treating an infection caused by NG. In a phase 2 study completed in 2017, which evaluated the efficacy, safety, and tolerability of gepotidacin in the treatment of uncomplicated urogenital gonorrhea caused by NG, gepotidacin was found to be efficacious against urogenital infection caused by NG (see Taylor SN, Morris DH, Avery AK, et al. Gepotidacin for the Treatment of Uncomplicated Urogenital Gonorrhea: A Phase 2, Randomized, Dose-Ranging, Single-Oral Dose Evaluation. Clin Infect Dis. 2018;67(4):504-512. doi:10.1093/cid/ciy145).

There is still a need for an improved treatment for infection caused by NG, in view of the emergence of drug-resistant forms of NG. As gepotidacin is a novel mechanism antibacterial compound, appropriate dosages and dosage regimens with acceptable efficacy and safety profiles are required.

### SUMMARY OF THE INVENTION

The references to methods of treatment in this description are to be interpreted as references to the compounds, pharmaceutical compositions and medicaments of the present invention for use in a method of treatment of the human (or animal) body by therapy.

In the first aspect, the present disclosure provides a method for treating an infection caused by *Neisseria gonorrhoeae* in a human in need thereof, comprising administering gepotidacin or a pharmaceutically acceptable salt thereof to said human, wherein the gepotidacin or a pharmaceutically acceptable salt thereof is administered orally in two doses of 3g each, within one day and wherein the Neisseria gonorrhea is resistant to ciprofloxacin.

In an embodiment, the present invention provides gepotidacin or a pharmaceutically acceptable salt thereof for use in the treatment of an infection caused by *Neisseria gonorrhoeae in a human in need thereof, comprising administering the gepotidacin or a phramaceutically acceptable salt thereof to said human,* wherein the gepotidacin or a pharmaceutically acceptable salt thereof is administered orally in two doses of 3g each, within one day and wherein the Neisseria gonorrhea is resistant to ciprofloxacin.

In another embodiment, the present invention provides gepotidacin or a pharmaceutically acceptable salt thereof for use in the treatment of an infection caused by Neisseria gonorrhoeae in a human in need thereof, comprising administering the gepotidacin or a pharmaceutically acceptable salt thereof to said human, wherein the gepotidacin or a pharmaceutically acceptable salt thereof is administered orally in two doses of 3g at an interval of 10-12 hours.

### DESCRIPTION OF DRAWINGS/FIGURES

Figure 1 shows the relationship between gepotidacin exposure and change in NG log₁₀ CFU/mL from baseline, as described in Example 2.
Figure 2 shows simulated gepotidacin PK curves for two single oral 3,000 mg doses, given 6 hours apart.
Figure 3 shows simulated gepotidacin PK curves for two single oral 3,000 mg doses, given 12 hours apart.

### DETAILED DESCRIPTION OF THE INVENTION

As used herein, the term "antibiotic" is synonymous with "antibacterial" and "antimicrobial". Gepotidacin and its racemic form are disclosed in WO 2008/1289422.

Gepotidacin is (2*R*)-2-({4-[(3,4-Dihydro-2*H*-pyrano[2,3-*c*]pyridin-6-ylmethyl)amino]-1-piperidinyl}methyl)-1,2-dihydro-3*H*,8*H*-2a,5,8a-triazaacenaphthylene-3,8-dione:

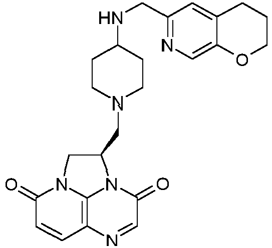

As used herein, the term "gepotidacin" may encompass gepotidacin free base, or a salt of gepotidacin. When a composition contains a salt of gepotidacin, the stated amount of gepotidacin in the composition refers to the amount of corresponding gepotidacin free base.

A lack of validated preclinical models for NG has prevented the establishment of the pharmacokinetic/pharmacodynamic (PK/PD) parameter and magnitude predictive of gepotidacin efficacy in an infection caused by NG. In the Phase 2 randomized dose-ranging single-oral dose evaluation, reported by Taylor SN et al., Clin Infect Dis. 2018;67(4):504-512, single oral doses of 1.5g and 3g of gepotidacin achieved ≥95% efficacy against urogenital infection caused by NG.

Surprisingly it has now been found that two 3g doses of gepotidacin administered within one day is a safe and efficacious treatment for infection caused by NG, with no increase in safety concerns. In particular, the present invention provides a treatment regimen of two 3g doses gepotidacin administered 6-12 hours apart, or 10-12 hours apart (total dose 6g within one day).

For the treatment of uncomplicated gonorrhea, both the CDC and WHO recommend a dual antibiotic regimen consisting of a single IM 250-mg dose of ceftriaxone and a single oral 1-g dose of azithromycin (Centers for Disease Control and Prevention (CDC). Sexually transmitted diseases treatment guidelines, 2015. MMWR Recomm Rep 2015;64(No. RR-3):1-137; and World Health Organization (WHO), Global priority list of antibiotic-resistant bacteria to guide research, discovery, and development of new antibiotics. 2017). The present invention advantageously provides a more convenient single drug treatment regimen.

### Method of Treatment

Thus in the first aspect, the present disclosure provides a method for treating an infection caused by NG in a human in need thereof, comprising administering gepotidacin or a pharmaceutically acceptable salt thereof to said human, wherein the gepotidacin or a pharmaceutically acceptable salt thereof is administered orally in two doses of 3g each, within one day and wherein the Neisseria gonorrhea is resistant to ciprofloxacin.

As would be understood by a skilled person, the two doses of 3 grams each provides a total dose of 6g in one day. "One day" is used herein to mean one period of 24 hours, which commences on taking the first dose. "Two doses within one day" is herein intended to mean the same as *"bis in die"* or "b.i.d."

In an embodiment, the present invention provides gepotidacin or a pharmaceutically acceptable salt thereof for use in the treatment of an infection caused by NG, *in a human in need thereof, comprising administering the gepotidacin or a phramaceutically acceptable salt thereof to said human* wherein the gepotidacin or a pharmaceutically acceptable salt thereof is administered orally in two doses of 3g each within one day and wherein the Neisseria gonorrhea is resistant to cirpofloxacin.

This specification discloses a method for treating an infection caused by NG in a human in need thereof, comprising administering gepotidacin or a pharmaceutically acceptable salt thereof to said human, wherein the gepotidacin or a pharmaceutically acceptable salt thereof is administered in two doses of 3g each at an interval of 6-12 hours .

This specification describes gepotidacin or a pharmaceutically acceptable salt thereof for use in the treatment of an infection caused by NG, wherein the gepotidacin or a pharmaceutically acceptable salt thereof is administered in two doses of 3g each at an interval of 6-12 hours.

In one aspect, the present disclosure provides a method for treating an infection caused by NG in a human in need thereof, comprising administering gepotidacin or a pharmaceutically acceptable salt thereof to said human, wherein the gepotidacin or a pharmaceutically acceptable salt thereof is administered orally in two doses of 3g each at an interval of 10-12 hours.

In one embodiment, the present invention provides gepotidacin or a pharmaceutically acceptable salt thereof for use in the treatment of an infection caused by NG in a human in need thereof, comprising administering the gepotidacin or a pharmaceutically acceptable salt thereof to said human wherein the gepotidacin or a pharmaceutically acceptable salt thereof is orally administered in two doses of 3g each at an interval of 10-12 hours.

As used herein, the phrase "infection caused by NG" may mean (1) that the skilled person suspects that the infection is caused by NG, for example due to clinical signs and symptoms, patient history or local epidemiology, or (2) that the skilled person proves or determines that the infection is caused by NG using standard methods such as culture results, or other information such as PCR, Gram stain or other stains. In one embodiment, in the method of the present invention, the infection is suspected to be caused by NG. In one embodiment, in the method of the present invention, the infection is proven to be caused by NG.

In one embodiment, for any aspect of the present invention, the gepotidacin is used for the treatment of an infection caused by a gepotidacin-susceptible isolate of NG.

As would be understood by the skilled person, "susceptible" means that an isolate of a microorganism is inhibited by the usually achievable concentration of an antimicrobial agent when the recommended dosage is used for the site of infection. Susceptibility to gepotidacin may be determined by the skilled person from an isolate recovered from a sample from an infected human, using standard methods as published by e.g. the United States Federal Drug Agency (Antibacterial Susceptibility Test Interpretive Criteria), Clinical and Laboratory Standards Institute ("CLSI" - see for example Performance Standards for Antimicrobial Susceptibility Testing. 29th ed. CLSI supplement M100. Wayne, PA: Clinical and Laboratory Standards Institute; 2019). or the European Union Committee on Antimicrobial Susceptibility Testing.

In one embodiment, "gepotidacin-susceptible isolate of NG "means that the gepotidacin MIC for an isolate of NG is 2 mg/L or less as measured by agar dilution according to CLSI guidelines. In one embodiment, it means that the MIC for the isolate is 1 mg/L or less as measured by agar dilution according to CLSI guidelines.

In one embodiment, for any aspect of the present invention, the human is aged 12, 13, 14, 15, 16 or 17 years old. In one embodiment, for any aspect of the present invention, the human is aged 18 years or above. In one embodiment, for any aspect of the present invention, the human is male. In one embodiment, for any aspect of the present invention, the human is female.

As used herein, an infection caused by NG in the urogenital area is termed "gonorrhea", which may include cervical and urethral gonorrhoea.

In one embodiment, for any aspect of the present invention, the infection caused by NG is a urogenital infection (also known as gonorrhea).

In one embodiment, for any aspect of the present invention, the infection caused by NG is a urethral or cervical infection (also known as urethral or cervical gonorrhea).

In one embodiment, for any aspect of the present invention, the urogenital infection caused by NG is an uncomplicated urethral or cervical infection (also known as uncomplicated urethral or cervical gonorrhea).

In one embodiment, for any aspect of the present invention, the infection caused by NG is a rectal infection.

In one embodiment, for any aspect of the present invention, the infection caused by NG is a pharyngeal infection. Infections of the pharynx caused by NG are more difficult to eradicate than infections at urogenital and anorectal sites. Gepotidacin has the advantage of favourable tissue distribution in the pharynx area in humans, as shown herein. Thus the present invention provides a method of treating a pharyngeal infection caused by NG in a human in need thereof, as defined herein.

In one embodiment, for any aspect of the present invention, the human has failed at least one prior line of treatment of the infection, such as by ceftriaxone or ciprofloxacin.

In one embodiment, for any aspect of the present invention, the NG is drug resistant. As used herein, "drug resistant" is synonymous with "non-susceptible" and means that an isolate is not inhibited by the usually achievable concentrations of antimicrobial agent when the recommended dosage is used for the site of infection. Drug resistance of a NG isolate may be suspected, for example through knowledge of the infected patient's medical history, e.g. recurrent urogenital infection, or may be proven through established techniques, which include phenotypic or genotypic determination. For example, a phenotypic test of drug resistance of a bacterium to an antibacterial agent may be performed by measuring susceptibility to the antibacterial agent, using standard methods and published breakpoints.

In one embodiment, in the present invention, "resistant" means resistant as defined by CLSI breakpoints. In one embodiment, "resistant" is as defined by the relevant breakpoint in M100 CLSI.

In one embodiment, for any aspect of the present invention, the NG isolate causing the infection is resistant to an antibacterial agent selected from the group consisting of ciprofloxacin, azithromycin, tetracycline, penicillin, ceftriaxone, cefixime, gentamicin and spectinomycin.

In one embodiment, for any aspect of the present invention, the NG isolate causing the infection is resistant to at least one antibacterial agent selected from ciprofloxacin, penicillin, and tetracycline.

In one embodiment, for any aspect of the present invention, the NG isolate is resistant to ciprofloxacin. In one embodiment, for any aspect of the present invention, the NG isolate is resistant to penicillin. In one embodiment, for any aspect of the present invention, the NG isolate is resistant to tetracycline.

In one embodiment, for any aspect of the present invention, the NG isolate causing the infection is resistant to two antibacterial agents selected from ciprofloxacin, penicillin and tetracycline.

In one embodiment, for any aspect of the present invention, the NG isolate causing the infection is resistant to ciprofloxacin, penicillin and tetracycline.

### Compounds of the invention

WO2008/128942 discloses the preparation of the free base and the hydrochloride salt of gepotidaci n.

It will be understood that the phrase "gepotidacin or a pharmaceutically acceptable salt thereof" is intended to encompass gepotidacin, a pharmaceutically acceptable salt of gepotidacin, a solvate of gepotidacin, or any pharmaceutically acceptable combination of these. Thus by way of non-limiting example used here for illustrative purpose, "gepotidacin or a pharmaceutically acceptable salt thereof" may include a pharmaceutically acceptable salt of gepotidacin that is further present as a solvate.

As used herein, the term "compound(s) of the invention" means a gepotidacin in any form, i.e., any salt or non-salt form (e.g., as a free base, or as a pharmaceutically acceptable salt thereof) and any physical form thereof (e.g., including non-solid forms (e.g., liquid or semi-solid forms), and solid forms (e.g., amorphous or crystalline forms, specific polymorphic forms, solvates, including hydrates), and mixtures of various forms.

Suitable pharmaceutically acceptable salts include those described by Berge, Bighley and Monkhouse, J. Pharm. Sci (1977) 66, pp 1-19.

Gepotidacin is a base (contains a basic moiety), and therefore a desired salt form may be prepared by any suitable method known in the art, including treatment of the free base with an inorganic acid, such as hydrochloric acid, hydrobromic acid, sulfuric acid, nitric acid, phosphoric acid, and the like, or with an organic acid, such as acetic acid, trifluoroacetic acid, maleic acid, succinic acid, mandelic acid, fumaric acid, malonic acid, pyruvic acid, oxalic acid, glycolic acid, salicylic acid, pyranosidyl acid, such as glucuronic acid or galacturonic acid, alpha-hydroxy acid, such as citric acid or tartaric acid, amino acid, such as aspartic acid or glutamic acid, aromatic acid, such as benzoic acid or cinnamic acid, sulfonic acid, such as p-toluenesulfonic acid, methanesulfonic acid, ethanesulfonic acid or the like. Examples of pharmaceutically acceptable salts include sulfates, pyrosulfates, bisulfates, sulfites, bisulfites, phosphates, chlorides, bromides, iodides, acetates, propionates, decanoates, caprylates, acrylates, formates, isobutyrates, caproates, heptanoates, propiolates, oxalates, malonates succinates, suberates, sebacates, fumarates, maleates, butyne-1,4-dioates, hexyne-1,6-dioates, benzoates, chlorobenzoates, methylbenzoates, dinitrobenzoates, hydroxybenzoates, methoxybenzoates, phthalates, phenylacetates, phenylpropionates, phenylbutrates, citrates, lactates, γ-hydroxybutyrates, glycollates, tartrates mandelates, and sulfonates, such as xylenesulfonates, methanesulfonates, propanesulfonates, naphthalene-1-sulfonates and naphthalene-2-sulfonates.

Pharmaceutically acceptable salts of gepotidacin include the acid addition salts, for example their salts with mineral acids e.g. hydrochloric, hydrobromic, sulphuric nitric or phosphoric acids, or organic acids, e.g. acetic, fumaric, succinic, maleic, citric, benzoic, p-toluenesulphonic, methanesulphonic, naphthalenesulphonic acid or tartaric acids.

The present invention includes within its scope all possible stoichiometric and non-stoichiometric salt forms.

In one embodiment, in any aspect of the present invention, the gepotidacin is gepotidacin free base.

In one embodiment, in any aspect of the present invention, the gepotidacin is gepotidacin methanesulphonate.

### Pharmaceutical Compositions And Formulations

Pharmaceutical compositions and formulations acceptable and adaptable for use in methods and/or uses of the present invention are prepared using conventional art known pharmaceutical compositions, formulation or chemical materials, formulary excipients, preparation means, processes and/or methods and conventional techniques, etc.

In particular, gepotidacin or pharmaceutically acceptable salts, used in the present invention may be formulated for administration in any convenient way for use in human or veterinary medicine, by analogy with other antibacterials/antitubercular compounds.

The pharmaceutical compositions used in the present disclosure may be formulated for administration by any route and include those in a form adapted for oral, topical or parenteral use and may be used in mammals including humans. According to the invention, gepotidacin or a pharmaceutically acceptable salt thereof is administered orally.

The compositions according to the disclosure may be in the form of tablets, capsules, powders, granules, lozenges, suppositories, creams or liquid preparations, such as oral or sterile parenteral solutions or suspensions.

In one embodiment, the gepotidacin or pharmaceutically acceptable salt thereof of the present invention is in a tablet or a capsule form. In one embodiment, it is in a tablet form. In one embodiment, the tablet is a 750mg tablet.

Tablets and capsules for oral administration in the present invention may be in unit dose presentation form, and may contain conventional excipients such as binding agents, fillers, tabletting lubricants, disintegrants, or wetting agents. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives, such as suspending agents, for example sorbitol, methyl cellulose, glucose syrup, gelatin, hydroxyethyl cellulose, carboxymethyl cellulose, aluminium stearate gel or hydrogenated edible fats, emulsifying agents, for example lecithin, sorbitan monooleate, or acacia; non-aqueous vehicles (which may include edible oils), for example almond oil, oily esters such as glycerine, propylene glycol, or ethyl alcohol; preservatives, for example methyl or propyl p-hydroxybenzoate or sorbic acid, and, if desired, conventional flavouring or colouring agents.

Suppositories (not part of the claimed invention) will contain conventional suppository bases, e.g. cocoa-butter or other glyceride.

For parenteral administration (not part of the claimed invention), fluid unit dosage forms are prepared utilizing the compound and a sterile vehicle, water being preferred. The compound, depending on the vehicle and concentration used, can be either suspended or dissolved in the vehicle. In preparing solutions the compound can be dissolved in water for injection and filter sterilised before filling into a suitable vial or ampoule and sealing.

Advantageously, agents such as a local anaesthetic, preservative and buffering agents can be dissolved in the vehicle. To enhance the stability, the composition can be frozen after filling into the vial and the water removed under vacuum. The dry lyophilized powder is then sealed in the vial and an accompanying vial of water for injection may be supplied to reconstitute the liquid prior to use. Parenteral suspensions are prepared in substantially the same manner except that the compound is suspended in the vehicle instead of being dissolved and sterilization cannot be accomplished by filtration. The compound can be sterilised by exposure to ethylene oxide before suspending in the sterile vehicle. Advantageously, a surfactant or wetting agent is included in the composition to facilitate uniform distribution of the compound.

Moreover, the quantity of the compound or pharmaceutical composition used in the present disclosure administered will vary depending on the patient and the mode of administration and can be any effective amount.

The compositions may contain from 0.1% by weight, preferably from 10-60% by weight, of the active material, depending on the method of administration. Where the compositions comprise dosage units, each unit will preferably contain from 50-1000 mg of the active ingredient. Unless otherwise noted, the amount of the active ingredient (i.e., gepotidacin) refers to that of gepotidacin free base.

Conventional administration methods may be suitable for use in the present disclosure.

Depending upon the treatment being effected, the compounds, and/or or compositions of the present disclosure can be administered orally, intravascularly, intraperitoneally, subcutaneously, intramuscularly or topically. Preferably, the composition is adapted for oral administration. For any aspect of the present invention, the gepotidacin or pharmaceutically acceptable salt thereof is administered orally.

The Examples set forth below are illustrative of the present invention and are not intended to limit, in any way, the scope of the present invention, which is as defined in the claims.

### EXAMPLES

### EXAMPLE 1 (reference example)

Gepotidacin exposures were assessed in plasma and other matrices, such as urine, saliva, epithelial lining fluid (ELF), Alveolar Macrophages (AM), cervix, rectum and pharynx, as follows:
1. Plasma, Epithelial Lining Fluid (ELF) and Alveolar Macrophage (AM) samples: A study to evaluate plasma and pulmonary pharmacokinetics (PK) following intravenous administration of gepotidacin in healthy adult subjects was conducted (see M Hossain, EF Dumont. Population Pharmacokinetic Modeling of Plasma and Epithelial Lining Fluid Data for a Novel Antimicrobial Compound. 54th Annual Interscience Conference on Antimicrobial Agents and Chemotherapy Meeting, 5-9 September 2014, Washington, D.C., USA).
2. Saliva, Urine and plasma samples:
   a. A single-dose study to assess the PK of oral gepotidacin in male and female adult participants with varying degrees of hepatic impairment and in matched control participants with normal hepatic function (J. Hands et al. Pharmacokinetics of Gepotidacin in Subjects with Hepatic Impairment. ID Week, 2-6 October 2019, Washington DC, USA)
   b. A single-dose study to assess the PK of gepotidacin in adult subjects with varying degrees of renal impairment and in matched control subjects with normal renal function
3. Urine, plasma, cervix, rectum and pharynx swabs:
   In a Phase 2a, single-center, single-arm, open-label study in the Unites States from July 2018 to January 2019 (see Overcash et al., Open Forum Infectious Diseases, Volume 6, Issue Supplement 2, October 2019, Page S539, https://doi.org/10.1093/ofid/ofz360.1343), a total of 22 female participants with uncomplicated Urinary Tract Infection (uUTI) were enrolled and evaluated for pharmacokinetics (PK), safety, and clinical efficacy. Participants were confined to the clinic from baseline (Days -1 to 1 predose) through on-therapy (Days 1 to 5) and returned as outpatients for test-of-cure (TOC; Days 10 to 13) and follow-up (Day 28t3). Participants received oral gepotidacin 1,500 mg twice daily (BID) for 5 days. Two participants (9%) withdrew from the study due to lost to follow-up and family reasons; there were no discontinuations due to adverse events (AEs). The majority of participants were white, age ranged from 19 to 60 years, and body mass index ranged from 20.9 to 37.9 kg/m². The number of past uUTI episodes ranged from 0 to 10 over the past 12 months across participants; the majority of participants reported ≤2 episodes. The mean total clinical symptom score at baseline was 7.9 (range: 4 to 12). All participants reported frequency and urgency, and all but 1 participant (5%) reported dysuria. Gepotidacin exposures in urine, cervix, rectum and pharynx were assessed.

### Results

Plasma, Epithelial Lining Fluid (ELF) and Alveolar Macrophage (AM) PK were assessed following a single IV infusion of 1000 mg gepotidacin over 2 hours. AM exposure was assessed by Cmax and AUC(0-12) and it was approximately 120-fold and 97-fold higher, respectively, than in ELF. AUC(0-12) ratio of AM and ELF to free plasma was 178 and 1.84, respectively (gepotidacin free fraction equal to 0.67). Concentrations in AM and ELF changed in a similar manner as in plasma over the 12-hour sampling period.

Saliva, Urine and Plasma concentrations were determined after administration of gepotidacin 750 mg IV (healthy and renally impaired subjects) and 1500 mg orally (healthy and hepatically impaired subjects, as well as uUTI patients). Saliva concentrations displayed a positive correlation to plasma concentrations in normal and renally impaired patients (R2 = 0.82), as well as in normal and hepatic impaired subjects (R2= 0.76). The ratio of saliva AUC to unbound plasma AUC was consistently close to unity (RAUC ≥ 0.75). In uUTI patients, urine Ctau exposures ranged from 322 to 352 µg/mL from Day 3 onwards. The minimum gepotidacin urine concentrations remained above a Minimum Inhibitory Concentration (MIC) of 4 µg/mL over the 12-hour dosing interval. Overall the gepotidacin urinary exposure (AUC[0-48]) in participants with moderate and severe hepatic impairment was approximately 3.2-fold and 3.9-fold greater than that observed in matched healthy controls, respectively.

In normal renal function subjects, 37.4% of the gepotidacin dose was removed in the urine (fe%). As expected, the amount decreased to 22.1% in moderate subjects and 7.9% in End-Stage Renal Disease (ESRD) subjects not on hemodialysis. Less than 2% of the gepotidacin dose was excreted in the urine for the ESRD subjects on hemodialysis.

Plasma, cervix, rectum and pharynx swabs were collected after administration of gepotidacin 1500 mg BID to uUTI patients. The rank order of gepotidacin free-drug concentrations was highest in rectal tissue; followed by cervical tissue, which was similar to plasma concentrations; followed by pharyngeal tissue. PK results are shown in Table 1.

**TABLE 1 Summary of gepotidacin cervical, rectal, pharyngeal, and free plasma concentration time data (µg/ml) (pharmacokinetic population)**

| **Matrix** | **N** | **Day** | **Planned relative time** | **n** | **Number imputed** | **Mean** | **%CV** | **Minimum** | **Maximum** |
|---|---|---|---|---|---|---|---|---|---|
| Cervical | 18 | 4 | Predose | 18 | 0 | 1.30 | 131.3 | 0.0601 | 5.94 |
| | | 4 | 2 hours postdose | 18 | 0 | 1.03 | 114.8 | 0.0190 | 4.25 |
| Rectal | 18 | 4 | Predose | 18 | 0 | 6.04 | 156.9 | 0.0678 | 36.30 |
| | | 4 | 2 hours postdose | 18 | 0 | 12.3 | 214.6 | 0.0989 | 90.90 |
| Pharyngeal | 20 | 4 | Predose | 19 | 0 | 0.0330 | 106.4 | 0.00275 | 0.165 |
| | | 4 | 2 hours postdose | 20 | 0 | 0.140 | 87.3 | 0.0118 | 0.527 |
| Free plasma | 21 | 4 | Predose | 21 | 0 | 0.618 | 44.2 | 0.308 | 1.33 |
| | | 4 | 2 hours postdose | 21 | 0 | 3.94 | 42.3 | 0.938 | 7.50 |
| ^{a}%CV, coefficient of variation; n, number of participants with evaluable values; N, number of participants in the treatment. | | | | | | | | | |

In conclusion, gepotidacin displays enhanced intracellular penetration (high AM levels when compared to plasma), supporting the development of this new antibacterial for intracellular infections, such as gonorrhea.

Saliva exposures correlated well with plasma, indicating that saliva samples could be collected in cases of limited feasibility of plasma samples. In addition, the high saliva levels supports the use of gepotidacin for the treatment of oral gonorrhea.

As the hepatic function decreased, the gepotidacin levels in urine increase, while with the reduction in renal function, reduced urine levels are observed.

Gepotidacin concentrations were observed in cervical, rectal, and pharyngeal swabs and support the use of gepotidacin for infections caused by NG in these areas.

### Example 2: PK/PD modelling

### (a) Hollow fiber infection model

A set of duplicate 7-day hollow fiber infection model studies was completed using an NG isolate from the Phase 2 clinical trial (as reported by Scangarella-Oman N et al., 2018. Antimicrob Agents Chemother 62:e01221-18. https://doi.org/10.1128/AAC.01221-18), with a gepotidacin MIC of 1 µg/mL and a ParC D86N mutation, to determine the gepotidacin exposure needed to prevent amplification of a resistant subpopulation when a pre-existing single step mutation to gepotidacin was already present. The results were discussed in VanScoy BD et al., A Hollow-Fiber Infection Model to Evaluate the Prevention of On- Therapy Resistance of Neisseria gonorrhoeae to Gepotidacin. 2018 STD Prevention Conference, 26-30 August 2018, Washington DC, USA.

### Method

The NG clinical isolate evaluated was known to be ciprofloxacin-resistant (minimum inhibitory concentration [MIC]= 2 mg/L), susceptible to ceftriaxone (MIC = 0.004 mg/L), and with a gepotidacin agar/broth MIC of 1/0.5 mg/L. The isolate was collected during the Phase 2 clinical study mentioned above, and contained the mutation, ParC D86N.

Gepotidacin, ciprofloxacin, and ceftriaxone MIC values were determined in triplicate using Gonococcal agar per CLSI guidelines (CLSI (2012) Methods for dilution antimicrobial susceptibility tests for bacteria that grow aerobically. Approved standard, 9th edition. CLSI document M07, Wayne, PA). In order to evaluate the MIC of each challenge compound under the liquid conditions utilized in the HFIM, MIC values were determined using fastidious broth (FB) medium modified to lack agarose.

10 mL of an initial bacterial density of 10⁶ colony forming units (CFU)/mL was inoculated into the hollow-fiber cartridge (Fibercell Systems, Frederick, MD), utilizing modified FB medium.

Assuming a 7-hour half-life for gepotidacin, human free-drug plasma concentration-time profiles were simulated following exposures observed after administration of 0.75 to 12 g of gepotidacin as a single oral dose. Ciprofloxacin and ceftriaxone exposures were simulated (half-lives of 3 and 7.5 hours, respectively), using free-drug plasma profiles following administration of a 0.5 g oral and 0.25 g intramuscular dose, respectively. Samples were collected for observation of simulated pharmacokinetic profiles and enumeration of bacterial burden over the study duration. All bacterial samples were plated on drug-free and agar supplemented with 2 x the agar MIC value of each respective challenge compound. MIC values were determined in duplicate for isolates that were found upon the drug-supplemented agar plates.

### Results

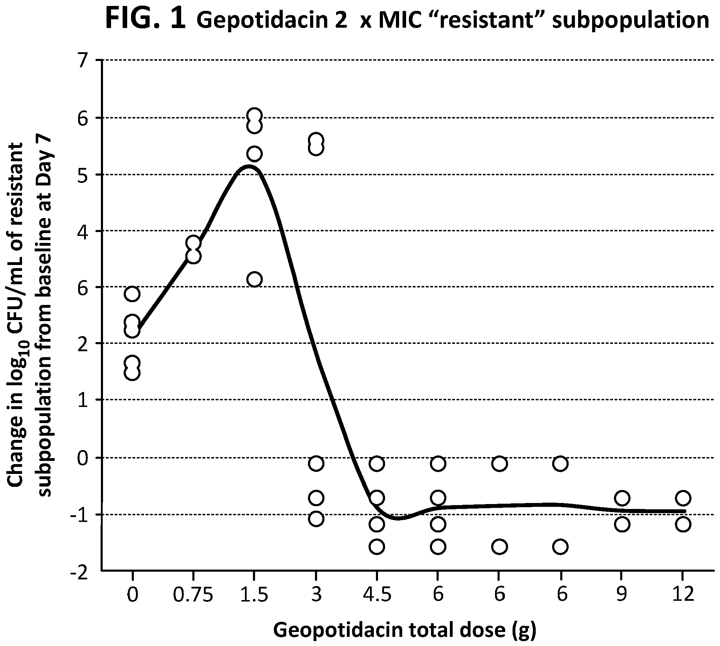

The NG isolate grew well in the hollow fibre infection model, reaching a total bacterial burden >8 log₁₀ CFU/mL by Day 1. The ciprofloxacin and ceftriaxone controls performed as expected given that the isolate was resistant to ciprofloxacin and susceptible to ceftriaxone. The gepotidacin exposures evaluated provided a full exposure response from treatment failure to success, with doses ≥ 4.5 g sterilizing the system over the seven-day period. The relationship between change in log₁₀ CFU/mL from bacterial burden of the gepotidacin-resistant subpopulation on Day 7 took on the form of an inverted-U, with doses ≥ 4.5 g preventing amplification of resistance within the system. Figure 1 shows the relationship between gepotidacin exposure (from 750 mg to 12 g) and change in log₁₀ CFU/mL from baseline of the gepotidacin-resistant subpopulation on Day 7.

In summary, total daily gepotidacin doses of equal to or greater than 4500 mg (including 2 gepotidacin 3g doses, administered either 8 or 12 hours apart) prevented resistance amplification to gepotidacin for NG in the hollow fiber infection model.

### (b) PK modelling

Population PK modelling (see Hossain M et al, Population Pharmacokinetic Modeling of First-Time-in-Human Data of GSK2140944, a Novel Antimicrobial Agent. 53nd Annual Interscience Conference on Antimicrobial Agents and Chemotherapy Meeting, 10-13 September 2013, Denver, CO, USA) and simulation using available pharmacokinetic data was conducted. PK simulations were conducted to assess safety with the objective of limiting the occurrence of maximum plasma exposures, which is important from a safety perspective due to corrected QT interval (QTc) increases and acetylcholinesterase inhibition.

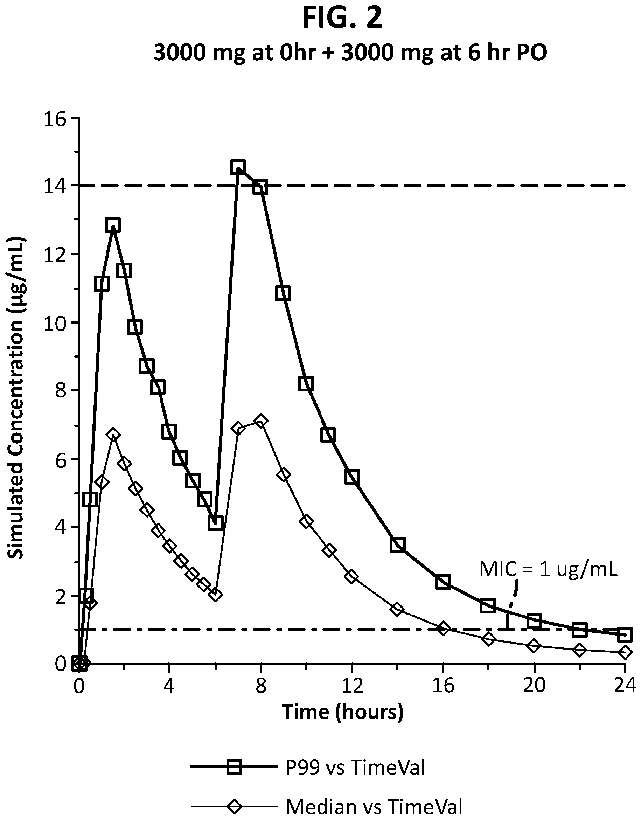

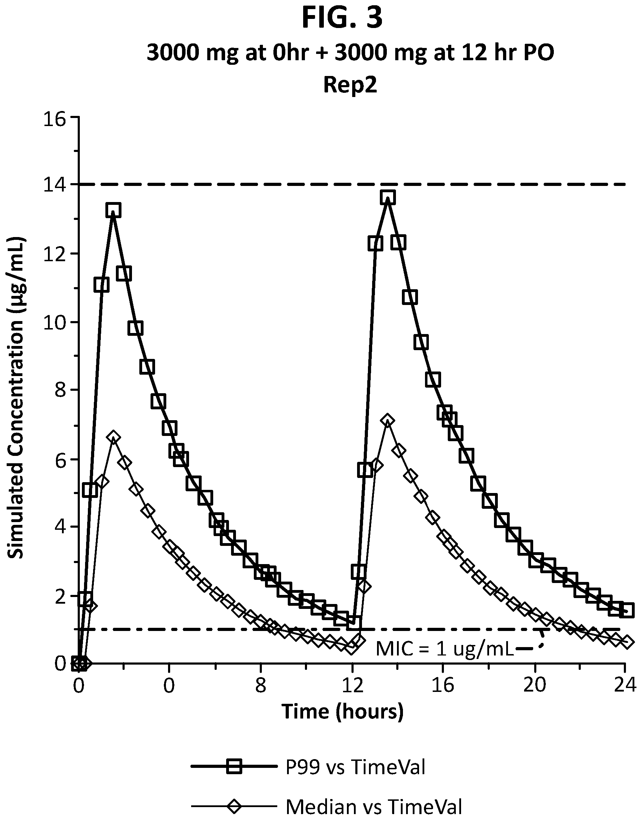

These simulations were conducted with the dose recommended for the treatment of gonorrhea. Figures 2 and 3 were generated based on simulations of gepotidacin administered as two single oral 3,000-mg doses, given either 6 or 12 hours apart (the dashed lines in Figures 2 and 3 represent the Cmax value of potential clinical concern). These simulations demonstrate that the majority of the subjects (> 97%) are below the level for the tested dosing intervals.

In conclusion, two 3g doses of gepotadicin, administered 6-12 hours apart, as opposed to the single 1.5g/3g dose used in the aforementioned Phase 2 trial, were found to have the advantages of:
- providing 2-fold higher systemic exposures that allow coverage of NG isolates with higher gepotidacin MIC values;
- reducing the risk of clinical resistance emergence; and
- maintaining maximum plasma concentrations below levels of potential safety concern (related to Cmax dependent changes in QTc interval and acetylcholinesterase effects).

### Example 3: Gepotadicin safety and pharmacokinetics (PK) in healthy adults and adolescents

Healthy adults and adolescents within the age ranges of 19-64 and 12-17 years old, respectively, participated in the study.

In Part 1 of the study, gepotidacin was administered with food to healthy adults (≥ 18 years) in period 1 (1500 mg oral single dose), followed by period 2 (3000 mg x 2 oral doses Q12h) and period 3 (3000 mg x 2 oral doses Q6h).

In Part 2 of the study, gepotidacin was administered with food to adolescents (12 to < 18 years) at the same dose regimens as period 1 (1500 mg oral single dose) and period 3 (3000 mg x 2 oral doses Q6h).

In Period 1, similar maximum concentration (Cmax) and slightly higher median area under the curve (AUC) exposures were observed for adolescents compared to adults. 1500 mg was generally well tolerated by adults and adolescents with few mild gastro-intestinal-tract (GI) adverse events (AEs).

In Period 2, the observed AUC0-t exposures following the first dose were similar in adults at the 3000 mg dose level Q12 and Q6h. Higher accumulation ratios were observed in adults for Q6h as compared to Q12h.

In Period 3, for the Q6h regimen following the second dose, the AUC was higher in adolescents, while Cmax was similar.

The shorter interval of Q6h resulted in higher accumulation ratios, as compared to Q12h, which led to a higher Cmax. The model had predicted 0.3% and 2.85 % of subjects above the value of potential clinical concern following the second dose of 3000mg Q12h and Q6h, respectively. The present study verified that a higher percentage of the subjects were above this value (15% for adults Q12h, 46% for adults Q6h and 58% for adolescents Q6h). No observed safety issues related to QT or AchE-I were observed with subjects having higher Cmax.

Projection of ΔΔQTc values based on this data indicated that a longer dosing window of 10-12 hours would have the benefit of maintaining sufficient levels of gepotidacin in the body for efficacy, with no changes to the overall risk/benefit balance.

### EXAMPLE 4: A Phase III, Randomized, Multicenter. Open-Label Study in Adolescent and Adult Participants Comparing the Efficacy and Safety of Gepotidacin to Ceftriaxone Plus Azithromycin in the Treatment of Uncomplicated Urogenital Gonorrhea Caused bv NG (BTZ116577)

This ongoing Phase III study aims to evaluate oral gepotidacin compared to intramuscular (IM) ceftriaxone plus oral azithromycin, a currently recommended treatment regimen, for the treatment of uncomplicated urogenital infection caused by NG in adolescent and adult participants.

### Overall Design:

This is a Phase III, open-label (sponsor-blinded), parallel-group, multicenter, comparator-controlled, noninferiority study in adolescent and adult participants comparing the efficacy and safety of oral gepotidacin to IM ceftriaxone plus oral azithromycin in the treatment of uncomplicated urogenital gonorrhea caused by NG. Participants will be stratified by gender and sexual orientation and age; and will be randomly assigned to receive either oral gepotidacin or IM ceftriaxone plus oral azithromycin. Appropriate safety and microbiological assessments will be conducted at the Baseline (Day 1) Visit and repeated at the TOC (Day 4 to 8) and Follow-up (Day 14 to 21) Visits. "Test-of-Cure" microbiological success will be defined by body site (i.e., urogenital and, as appropriate, pharyngeal and/or rectal) as culture-confirmed bacterial eradication of NG observed at the TOC (Day 4 to 8) Visit.

### Number of Participants:

Approximately 500 to 600 participants will be screened and randomized to achieve approximately 400 participants with culture-confirmed urogenital gonorrhea in the Microbiological Intent-to-Treat (micro-ITT) Population, for an estimated total of 200 participants per treatment group in the micro-ITT Population. Enrollment will continue until the target number of participants in the micro-ITT Population has been reached.

### Treatment Groups and Duration:

Participants will receive one of the following treatments:
1. Gepotidacin: 3000 mg administered orally at the study site during the Baseline (Day 1) Visit followed by self-administration of a second oral 3000-mg dose as an outpatient 6 to 12 hours after the first dose (optimum window is 8 to 10 hours after the first dose; however, for participants who weigh <50 kg or have moderate renal impairment, the second dose should be taken approximately 12 hours after the first dose).
2. Ceftriaxone plus azithromycin: A single IM 500-mg dose of ceftriaxone plus a single oral 1-g dose of azithromycin administered at the study site during the Baseline (Day 1) Visit.

The study duration is approximately 21 days with 3 planned study visits: Baseline (Day 1) Visit, TOC (Day 4 to 8) Visit, and Follow-up (Day 14 to 21) Visit.

### Dose Justification

In the Phase II study, both single oral doses of 1500 and 3000 mg gepotidacin were ≥95% efficacious (bacterial eradication) against urogenital NG with no unexpected safety signals at either dose. A total of 3 participants failed therapy at the urogenital body site (all with a baseline gepotidacin MIC of 1 pg/mL, which was among the highest MIC for gepotidacin), and analysis of the posttreatment cultures identified 2 isolates that had become resistant to gepotidacin (TOC gepotidacin MICs ≥32 µg/mL). Further analysis revealed that both isolates also had a pre-existing D86N polymorphism in the topoisomerase IV gene at Baseline. Following treatment, both isolates showed a second mutation, an A92T substitution in the DNA gyrase enzyme, which resulted in reduced susceptibility to gepotidacin. Of the 8 participants in the study with D86N-positive baseline urogenital NG isolates, 5 were microbiological successes (bacterial eradication), including 2 that were infected with baseline organisms with the higher gepotidacin MIC of 1 µg/mL. These data suggest that the 3000-mg single dose may provide close to an effective exposure and that this genotype may be overcome with appropriate dosing. In the current study, gepotidacin will be administered as 2 oral 3000-mg doses.

The two 3000-mg doses, administered 6 to 12 hours apart, compared with the single 3000-mg dose used in the Phase II study, is expected to provide 2-fold higher systemic exposures that allow coverage of NG isolates with higher gepotidacin MIC values, which are likely to be observed in this global Phase III study, and also to reduce the risk of resistance emergence.

## Claims

1. Gepotidacin or a pharmaceutically acceptable salt thereof for use in the treatment of an infection caused by *Neisseria gonorrhoeae* in a human in need thereof, comprising administering the gepotidacin or a pharmaceutically acceptable salt thereof to said human, wherein the gepotidacin or a pharmaceutically acceptable salt thereof is administered orally in two doses of 3g each within one day; and wherein the *Neisseria gonorrhoeae* isolate is resistant to ciprofloxacin.

2. Gepotidacin or a pharmaceutically acceptable salt thereof for use in the treatment of an infection caused by *Neisseria gonorrhoeae* in a human in need thereof, comprising administering the gepotidacin or a pharmaceutically acceptable salt thereof to said human, wherein the gepotidacin or a pharmaceutically acceptable salt thereof is administered orally in two doses of 3g at an interval of 10-12 hours.

3. Gepotidacin or a pharmaceutically acceptable salt thereof for use according to claim 2, wherein the human has failed at least one prior line of treatment of the infection.

4. Gepotidacin or a pharmaceutically acceptable salt thereof for use according to claim 2, wherein the *Neisseria gonorrhoeae* isolate is resistant to an antibacterial agent selected from the group consisting of: ciprofloxacin, azithromycin, tetracyline, penicillin, ceftriaxone, cefixime, gentamicin and spectinomycin.

5. Gepotidacin or a pharmaceutically acceptable salt thereof for use according to any of claims 1-4, wherein the gepotidacin is gepotidacin free base.

6. Gepotidacin or a pharmaceutically acceptable salt thereof for use according to any of claims 1-4, wherein the gepotidacin is gepotidacin methanesulphonate.

7. Gepotidacin or a pharmaceutically acceptable salt thereof for use according to any of claims 1-6, wherein the human is aged 12, 13, 14, 15, 16 or 17 years old.

8. Gepotidacin or a pharmaceutically acceptable salt thereof for use according to any of claims 1-6, wherein the human is aged 18 years or above.

9. Gepotidacin or a pharmaceutically acceptable salt thereof for use according to any of claims 1-8, wherein the human is male.

10. Gepotidacin or a pharmaceutically acceptable salt thereof for use according to any of claims 1-8, wherein the human is female.

11. Gepotidacin or a pharmaceutically acceptable salt thereof for use according to any of claims 1-10, wherein the infection caused by *Neisseria gonorrhoeae* is a urogenital infection.

12. Gepotidacin or a pharmaceutically acceptable salt thereof for use according to claim 11, wherein the infection caused by *Neisseria gonorrhoeae* is a urethral or cervical infection.

13. Gepotidacin or a pharmaceutically acceptable salt thereof for use according to claim 12, wherein the urogenital infection caused by *Neisseria gonorrhoeae* is an uncomplicated urethral or cervical infection.

14. Gepotidacin or a pharmaceutically acceptable salt thereof for use according to any of claims 1-10, wherein the infection caused by *Neisseria gonorrhoeae* is a rectal infection.

15. Gepotidacin or a pharmaceutically acceptable salt thereof for use according to any of claims 1-10, wherein the infection caused by *Neisseria gonorrhoeae* is a pharyngeal infection.

## Patentansprüche

1. Gepotidacin oder ein pharmazeutisch akzeptables Salz davon zur Verwendung bei der Behandlung einer durch *Neisseria gonorrhoeae* verursachten Infektion bei einem Menschen, der dessen bedarf, umfassend
Verabreichen des Gepotidacins oder eines pharmazeutisch akzeptablen Salzes davon an den Menschen,
wobei das Gepotidacin oder ein pharmazeutisch akzeptables Salz davon oral in zwei Dosen von jeweils 3g innerhalb eines Tages verabreicht wird; und wobei das *Neisseria gonorrhoeae*-Isolat resistent gegen Ciprofloxacin ist.

2. Gepotidacin oder ein pharmazeutisch akzeptables Salz davon zur Verwendung bei der Behandlung einer durch *Neisseria gonorrhoeae* verursachten Infektion bei einem Menschen, der dessen bedarf, umfassend
Verabreichen des Gepotidacins oder eines pharmazeutisch akzeptablen Salzes davon an den Menschen,
wobei das Gepotidacin oder ein pharmazeutisch akzeptables Salz davon oral in zwei Dosen von 3g in einem Abstand von 10-12 Stunden verabreicht wird.

3. Gepotidacin oder ein pharmazeutisch akzeptables Salz davon zur Verwendung gemäß Anspruch 2,
wobei bei dem Menschen zumindest eine vorherige Behandlungslinie der Infektion versagt hat.

4. Gepotidacin oder ein pharmazeutisch akzeptables Salz davon zur Verwendung gemäß Anspruch 2,
wobei das *Neisseria gonorrhoeae*-Isolat resistent ist gegen ein antibakterielles Mittel, das ausgewählt ist aus einer Gruppe bestehend aus Ciprofloxacin, Azithromycin, Tetracylin, Penicillin, Ceftriaxon, Cefixim, Gentamicin und Spectinomycin.

5. Gepotidacin oder ein pharmazeutisch akzeptables Salz davon zur Verwendung gemäß mindestens einem der Ansprüche 1-4, wobei das Gepotidacin die freie Base von Gepotidacin ist.

6. Gepotidacin oder ein pharmazeutisch akzeptables Salz davon zur Verwendung gemäß mindestens einem der Ansprüche 1-4, wobei das Gepotidacin ein Gepotidacin-Methansulfonat ist.

7. Gepotidacin oder ein pharmazeutisch akzeptables Salz davon zur Verwendung gemäß mindestens einem der Ansprüche 1-6, wobei der Mensch 12, 13, 14, 15, 16 oder 17 Jahre alt ist.

8. Gepotidacin oder ein pharmazeutisch akzeptables Salz davon zur Verwendung gemäß mindestens einem der Ansprüche 1-6, wobei der Mensch 18 Jahre oder älter ist.

9. Gepotidacin oder ein pharmazeutisch akzeptables Salz davon zur Verwendung gemäß mindestens einem der Ansprüche 1-8, wobei der Mensch männlich ist.

10. Gepotidacin oder ein pharmazeutisch akzeptables Salz davon zur Verwendung gemäß mindestens einem der Ansprüche 1-8, wobei der Mensch weiblich ist.

11. Gepotidacin oder ein pharmazeutisch akzeptables Salz davon zur Verwendung gemäß mindestens einem der Ansprüche 1-10, wobei die durch *Neisseria gonorrhoeae* verursachte Infektion eine urogenitale Infektion ist.

12. Gepotidacin oder ein pharmazeutisch akzeptables Salz davon zur Verwendung gemäß Anspruch 11, wobei die durch *Neisseria gonorrhoeae* verursachte Infektion eine urethrale oder zervikale Infektion ist.

13. Gepotidacin oder ein pharmazeutisch akzeptables Salz davon zur Verwendung gemäß Anspruch 12, wobei die durch *Neisseria gonorrhoeae* verursachte urogenitale Infektion eine unkomplizierte urethrale oder zervikale Infektion ist.

14. Gepotidacin oder ein pharmazeutisch akzeptables Salz davon zur Verwendung gemäß mindestens einem der Ansprüche 1-10, wobei die durch *Neisseria gonorrhoeae* verursachte Infektion eine rektale Infektion ist.

15. Gepotidacin oder ein pharmazeutisch akzeptables Salz davon zur Verwendung gemäß mindestens einem der Ansprüche 1-10, wobei die durch *Neisseria gonorrhoeae* verursachte Infektion eine pharyngeale Infektion ist.

## Revendications

1. Gépotidacine ou un sel pharmaceutiquement acceptable de celle-ci pour une utilisation dans le traitement d'une infection causée par *Neisseria gonorrhoeae* chez un humain qui en a besoin, comprenant l'administration de la gépotidacine ou d'un sel pharmaceutiquement acceptable de celle-ci audit humain, où la gépotidacine ou un sel pharmaceutiquement acceptable de celle-ci est administrée par voie orale en deux doses de 3 g chacune en un jour; et où l'isolat de *Neisseria gonorrhoeae* est résistant à la ciprofloxacine.

2. Gépotidacine ou un sel pharmaceutiquement acceptable de celle-ci pour une utilisation dans le traitement d'une infection causée par *Neisseria gonorrhoeae* chez un humain qui en a besoin, comprenant l'administration de la gépotidacine ou d'un sel pharmaceutiquement acceptable de celle-ci audit humain, où la gépotidacine ou un sel pharmaceutiquement acceptable de celle-ci est administrée par voie orale en deux doses de 3 g à un intervalle de 10 à 12 heures.

3. Gépotidacine ou un sel pharmaceutiquement acceptable de celle-ci pour une utilisation selon la revendication 2, où l'être humain n'a pas répondu à au moins une ligne de traitement de l'infection antérieure.

4. Gépotidacine ou un sel pharmaceutiquement acceptable de celle-ci pour une utilisation selon la revendication 2, où l'isolat de *Neisseria gonorrhoeae* est résistant à un agent antibactérien choisi dans le groupe consistant en: ciprofloxacine, azithromycine, tétracycline, pénicilline, céftriaxone, céfixime, gentamicine et spectinomycine.

5. Gépotidacine ou un sel pharmaceutiquement acceptable de celle-ci pour une utilisation selon l'une quelconque des revendications 1 à 4, où la gépotidacine est la base libre de gépotidacine.

6. Gépotidacine ou un sel pharmaceutiquement acceptable de celle-ci pour une utilisation selon l'une quelconque des revendications 1 à 4, où la gépotidacine est le méthanesulfonate de gépotidacine.

7. Gépotidacine ou un sel pharmaceutiquement acceptable de celle-ci pour une utilisation selon l'une quelconque des revendications 1 à 6, où l'humain est âgé de 12, 13, 14, 15, 16 ou 17 ans.

8. Gépotidacine ou un sel pharmaceutiquement acceptable de celle-ci pour une utilisation selon l'une quelconque des revendications 1 à 6, où l'humain est âgé de 18 ans ou plus.

9. Gépotidacine ou un sel pharmaceutiquement acceptable de celle-ci pour une utilisation selon l'une quelconque des revendications 1 à 8, où l'humain est de sexe masculin.

10. Gépotidacine ou un sel pharmaceutiquement acceptable de celle-ci pour une utilisation selon l'une quelconque des revendications 1 à 8, où l'humain est de sexe féminin.

11. Gépotidacine ou un sel pharmaceutiquement acceptable de celle-ci pour une utilisation selon l'une quelconque des revendications 1 à 10, où l'infection causée par *Neisseria gonorrhoeae* est une infection urogénitale.

12. Gépotidacine ou un sel pharmaceutiquement acceptable de celle-ci pour une utilisation selon la revendication 11, où l'infection causée par *Neisseria gonorrhoeae* est une infection urétrale ou cervicale.

13. Gépotidacine ou un sel pharmaceutiquement acceptable de celle-ci pour une utilisation selon la revendication 12, où l'infection urogénitale causée par *Neisseria gonorrhoeae* est une infection urétrale ou cervicale non compliquée.

14. Gépotidacine ou un sel pharmaceutiquement acceptable de celle-ci pour une utilisation selon l'une quelconque des revendications 1 à 10, où l'infection causée par *Neisseria gonorrhoeae* est une infection rectale.

15. Gépotidacine ou un sel pharmaceutiquement acceptable de celle-ci pour une utilisation selon l'une quelconque des revendications 1 à 10, où l'infection causée par *Neisseria gonorrhoeae* est une infection pharyngée.
